# EUROPEAN PATENT APPLICATION

(11) **EP 3 770 249 A1**
(43) Date of publication of application: **27.01.2021**
(21) Application number: 19770977.7
(22) Date of filing: 20.03.2019
(51) Int. Cl.: C12N 5/00, C12N 5/02

(54) **METHOD FOR THREE-DIMENSIONALLY CULTURING PLURIPOTENT STEM CELLS**

(30) Priority: 20.03.2018 JP 2018052656
(71) Applicant: Fujifilm Corporation, Minato-ku Tokyo 106-8620 (JP)
(72) Inventor: MURAKAMI, Yuta, Ashigarakami-gun, Kanagawa 258-8577 (JP)
(74) Representative: Hoffmann Eitle
(86) International application number: PCT/JP2019/011644
(87) International publication number: WO 2019/181999

(57) **Abstract**

An object of the present invention is to provide a method for three-dimensionally culturing a pluripotent stem cell, in which the pluripotent stem cell can maintain good proliferative properties even in a case where the frequency of the medium exchange is reduced. According to the present invention, a method for three-dimensionally culturing a pluripotent stem cell, in which a pluripotent stem cell is cultured in a medium that always contains 35 ng/mL or more of basic fibroblast growth factor until a cell concentration reaches 1.4 × 10⁶ cells/mL or more, is provided.

## Description

### Field of the Invention

The present invention relates to a method for three-dimensionally culturing a pluripotent stem cell.

### Description of the Related Art

As pluripotent stem cells, an induced pluripotent stem cell (also referred to as an iPS cell), an embryonic stem cell (also referred to as an ES cell), and the like are known. In the field of regenerative medicine, research for the practical application of the iPS cells has been particularly advanced.

For the practical application of pluripotent stem cells, it is important to establish a three-dimensional large-scale culturing system for pluripotent stem cells. Non-Patent Document 1 discloses that, by using a specific three-dimensional suspension spinner culturing device (bioreactor), setting an appropriate stirring blade rotation speed, an pH, and a dissolved oxygen concentration, a human iPS cell maintained on mouse embryonic fibroblasts (MEFs) or SNL feeder cells and on Matrigel, vitronectin, and a laminin E8 fragment could be amplified to about 5 times, up to 1 x 10⁸ cells of undifferentiated cells per 100 mL culture tank, in 4 days through the formation of cell aggregates by culturing from a single cell suspension using mTeSR1 (registered trademark), Essential 8 (trade name), and StemFit (registered trademark) AK03, without depending on the type of the establishment method using retrovirus vector or episomal vector.

In addition, Patent Document 1 discloses a serum-free and xeno-free medium which contains ascorbic acid, basic fibroblast growth factor (bFGF), a xeno-free serum replacement, and a lipid mixture, in which pluripotent stem cells are maintained in an undifferentiated state in the absence of feeder cell support. Patent Document 2 discloses a method for growing and maintaining the above pluripotent stem cells in an undifferentiated state, including a step of culturing pluripotent stem cells in the above medium. Further, Patent Document 2 discloses a serum-free and xeno-free medium which includes basic fibroblast growth factor (bFGF), transforming growth factor β-3 (TGFβ3), and ascorbic acid, in which the concentration of the ascorbic acid in the medium is at least about 50 µg/ml, and the medium is used for maintaining pluripotent stem cells in an undifferentiated state in the absence of feeder cell support. Patent Document 2 discloses a method for growing and maintaining the above pluripotent stem cells in an undifferentiated state, including a step of culturing pluripotent stem cells in the above medium. Patent Document 3 discloses a three-dimensional spherical culturing system including a functional polymer for large-scale production of human pluripotent stem cells. In addition, Patent Document 4 discloses a temperature stable mutant of fibroblast growth factor.

### Prior Art Documents

### Patent Documents

Patent Document 1: JP2013-510567A
Patent Document 2: JP2017-225456A
Patent Document 3: JP2015-530104A
Patent Document 4: JP2014-507951A

### Non-Patent Documents

Non-Patent Document 1: Biotechnology, 2014, Vol. 92, No. 9, pp. 483-486

### SUMMARY OF THE INVENTION

In a case where pluripotent stem cells are cultured three-dimensionally in a large scale, a method in which the culture medium is exchanged to or added with a complete medium as the number of cells increases is generally used. The complete medium is a medium in which pluripotent stem cells can be unassistedly proliferated and maintained. However, since the medium for culturing pluripotent stem cells is expensive, the above method takes a high cost. As a solution to this problem, the cost reduction is performed by adding only the components consumed by the cells. That is, in this culturing method, a seed medium at the time of cell seeding and a feed medium supplemented after the next day of the seeding are used in combination. However, in some cases, it was insufficient as a means for three-dimensionally culturing pluripotent stem cells to a high cell concentration.

An object to be achieved in the present invention is to provide a method for three-dimensionally culturing a pluripotent stem cell, in which the pluripotent stem cell can maintain good proliferative properties even in a case where the frequency of the medium exchange or medium addition is reduced.

The present inventors performed extensive studies to solve the above-described problems and, as a result, have found that, in case of three-dimensionally culturing pluripotent stem cells, pluripotent stem cells can be three-dimensionally cultured while maintaining good proliferative properties of the pluripotent stem cells by culturing the pluripotent stem cells in a medium that always contains 35 ng/mL or more of basic fibroblast growth factor (bFGF) until a cell concentration reaches 1.4 × 10⁶ cells/mL or more. The present invention has been completed based on the above findings.

That is, according to an aspect of the present invention, the following inventions are provided.
(1) A method for three-dimensionally culturing a pluripotent stem cell,
   in which a pluripotent stem cell is cultured in a medium that always contains 35 ng/mL or more of basic fibroblast growth factor until a cell concentration reaches 1.4 × 10⁶ cells/mL or more.
(2) The method according to (1), in which the basic fibroblast growth factor includes a temperature stable basic fibroblast growth factor.
(3) The method according to (1) or (2), in which a concentration of the basic fibroblast growth factor during culturing is maintained at 40% or more of a concentration of the basic fibroblast growth factor at a start of the culturing.
(4) The method according to any one of (1) to (3), in which a medium or a specific medium component is exchanged or added at intervals of 2 days or more.
(5) The method according to any one of (1) to (4), in which a cell concentration at a start of three-dimensional culturing is 1 × 10⁴ cells/mL or more and 1 × 10⁶ cells/mL or less.
(6) The method according to any one of (1) to (5), in which the number of cells 4 days after a start of culturing is 5 times or more the number of cells at the start of the culturing.
(7) The method according to any one of (1) to (6), in which the pluripotent stem cell is proliferated while maintaining pluripotency.

According to a method for three-dimensionally culturing a pluripotent stem cell according to the present invention, a pluripotent stem cell can maintain good proliferative properties even in a case where the frequency of the medium exchange is reduced.

### BRIEF DESCRIPTION OF THE DRAWINGS

Fig. 1 is a graph showing a relationship between culturing days and proliferation rates of cells.
Fig. 2 is a graph showing a relationship between culturing days and concentrations of bFGF in a medium.

### DESCRIPTION OF THE PREFERRED EMBODIMENTS

Hereinafter, embodiments for performing the present invention will be described in detail.

A method for three-dimensionally culturing a pluripotent stem cell according to the embodiment of the present invention is a method in which a pluripotent stem cell is cultured in a medium that always contains 35 ng/mL or more of basic fibroblast growth factor until a cell concentration reaches 1.4 × 10⁶ cells/mL or more.

### <Pluripotent stem cell>

A pluripotent stem cell refers to a cell that has the ability to differentiate into all three germ layers of endoderm, ectoderm, and mesoderm.

The pluripotent stem cell includes an embryonic stem cell (ESC) and an induced pluripotent stem cell (iPS cell). As the pluripotent stem cell, an induced pluripotent stem cell (iPS cells) is preferable.

In addition, as the pluripotent stem cell, an embryonic stem cell derived from humans or primates (for example, monkeys) is preferable, and a human pluripotent stem cell is more preferable.

As the embryonic stem cell (ESC) and the induced pluripotent stem cell (iPS cell), a human embryonic stem cell and a human induced pluripotent stem cell are preferably used.

Embryonic stem cells include a cell obtained from an embryonic tissue formed after pregnancy (for example, a preimplantation blastocyst), expanded blastocyst cell (EBC) obtained from a blastocyst of late implantation or early gastrulation, and an embryonic germ (EG) cell obtained from a fetal genital tissue at any time during pregnancy, preferably prior to 10 weeks of pregnancy.

An embryonic stem cell can be obtained by well-known methods. For example, a human embryonic stem cell can be isolated from a human blastocyst. A human blastocyst is obtained from a human preimplantation embryo or an in vitro fertilization (IVF) embryo. Alternatively, a single-cell human embryo can be grown to the blastocyst stage. An expanded blastocyst cell (EBC) is available from a blastocyst at an early stage of gastrulation at least 9 days after fertilization. In addition, an embryonic germ (EG) cell can be prepared from a primordial germ cell obtained from a fetus at about 8 to 11 weeks after pregnancy in the case of a human fetus.

It is also possible to use a commercially available embryonic stem cell. Human embryonic stem cells can be purchased from, for example, the National Institutes of Health (NIH) human embryonic stem cell registry (www.escr.nih.gov).

An induced pluripotent stem (iPS) cell is a pluripotent stem cell obtained by introducing a reprogramming factor into a somatic cell.

The somatic cell is not particularly limited, and any somatic cell can be used. For example, in addition to somatic cells in the fetal period, mature somatic cells may be used. Examples of the somatic cells include: (1) tissue stem cells (somatic stem cells) such as a neural stem cell, a hematopoietic stem cell, a mesenchymal stem cell, and a dental pulp stem cell; (2) a tissue progenitor cells; and (3) differentiated cells such as a fibroblast (a skin cell and the like), an epithelial cell, a hepatocyte, a lymphocyte (T cell or B cell), an endothelial cell, a muscle cell, a hair cell, a gastric mucosal cell, an intestinal cell, a splenocyte, a pancreatic cell (an exocrine pancreatic cell and the like), a brain cell, a lung cell, a kidney cell, and a skin cell.

The living body from which the somatic cells are derived is not particularly limited, and examples thereof include, a human and a non-human animal (for example, a monkey, a sheep, a cow, a horse, a dog, a cat, a rabbit, a rat, and a mouse). The living body is preferably a human.

The reprogramming factor to be introduced into a somatic cell is not particularly limited. Examples thereof include Oct3/4, Klf4, c-Myc, Sox2, Nanog, Klf2, L-Myc, N-Myc, Klf5, Lin28, Tert, Fbx15, ERas, ECAT15-1, ECAT15-2, Tcl1, β-catenin, ECAT1, Esg1, Dnmt3L, ECAT8, Gdf3, Sox15, Fthl17, Sal14, Rex1, UTF1, Stella, Stat3, Grb2, Prdm14, Nr5a1, Nr5a2, and E-cadherin. Here, two or more genes can be randomly selected from these gene groups and introduced in any combination. Among these, a combination having at least Oct3/4, Sox2, Klf4, and c-Myc, a combination having at least Oct3/4, Sox2, Klf4, and L-Myc, or a combination having at least Oct3/4, Sox2, Nanog, and Lin28 preferable.

In addition, a gene to be introduced and a cell to which the gene is introduced is preferably derived from the same species. For example, a gene to be introduced into a human-derived cell is preferably a human gene. Examples of genes to be introduced into the human-derived somatic cell preferably include a combination having at least human Oct3/4, human Sox2, human Klf4, and human c-Myc, a combination having at least human Oct3/4, human Sox2, human Klf4, and human L-Myc, and a combination having at least human Oct3/4, human Sox2, human Nanog, and human Lin28.

The gene as the reprogramming factor can be introduced into a somatic cell using a gene expression vector. The gene expression vector is not particularly limited, and examples thereof include a virus vector, a plasmid vector, an artificial chromosome vector, and a transposon vector. Examples of the virus vector include a retrovirus vector, an adenovirus vector, a Sendai virus vector, a lentivirus vector, and an adeno-associated virus vector.

An undifferentiated cell obtained by introducing a reprogramming factor into a somatic cell and having a relatively low ability to differentiate into a specific differentiated cell may be prepared in-house by introducing a reprogramming factor into a somatic cell. Alternatively, cells provided or sold by a research institution or a company may be obtained. That is, the first step of the embodiment of the present invention may be a step of obtaining an induced pluripotent stem cell from an induced pluripotent stem cell bank.

For example, an iPS cell provided by FUJIFILM Cellular Dynamics, Inc. can be obtained and used.

In addition, 201B7, 253G1, 253G4, 1201C1, 1205D1, 1210B2, 1231A3, 1383D2, 1383D6, iPS-TIG120-3f7, iPS-TIG120-4f1, iPS-TIG114-4f1, CiRA086Ai-m1, CiRA188Ai-M1, or iRA188Ai-W1 provided by Kyoto University iPS Cell Research Institute can be obtained and used.

In addition, cells are available from the iPS cell Bank which are constructed by National Institutes of Health (NIH), California Institute of Regenerative Medicine, New York Stem Cell Foundation, and European Bank for induced Pluripotent Stem cells and the like.

### <Three-dimensional culturing>

The three-dimensional culturing is a method for three-dimensionally culturing a cell after seeding and is different from two-dimensional culturing in which a cell is cultured by attaching the cell on a flat surface of a vessel such as a petri dish. In three-dimensional culturing, cells can spontaneously form a three-dimensional cell aggregate or a spheroid, but a method using an extrinsic matrix such as an extracellular matrix or a microcarrier can also be used. In the three-dimensional culturing, the cell aggregate is suspended in a liquid medium. Suspension culturing is a culturing in which pluripotent stem cells are suspended in a medium rather than attached to the surface of a substrate. That is, in the suspension culturing, the medium may be stirred to acquiring the suspension force, or a polymer for acquiring the suspension force may be added to the culture medium. For example, JP2015-530104A discloses a method for three-dimensionally culturing a pluripotent stem cell in a medium containing cell source nanofibrils without stirring.

### <Basic fibroblast growth factor>

In the present invention, a pluripotent stem cell is cultured in a medium that always contains 35 ng/mL or more of basic fibroblast growth factor.

Basic fibroblast growth factor (sometimes referred to as bFGF, FGF2, or FGF-β) is a member of the fibroblast growth factor family.

Basic fibroblast growth factor (also known as bFGF, FGF2, or FGF-β) is a kind of cytokine belonging to the fibroblast growth factor family and has functions such as proliferation and angiogenesis of fibroblast during wounding. In the present invention, basic fibroblast growth factor has an action of contributing to the maintenance of the undifferentiation of cells. The bFGF used in the present invention may be a naturally occurring bFGF, a chemically synthesized bFGF, or a genetically modified bFGF. For example, the amino acid sequence and the base sequence of human bFGF are registered under a GenBank accession number NP_001997.5 and a GenBank accession number NM_002006.4. As bFGF, various commercial products can also be used.

The medium used in the present invention will be described later. In a case where a commercially available medium containing bFGF in advance is used, such a commercially available medium may be used as it is.

In the present invention, the basic fibroblast growth factor includes a temperature stable basic fibroblast growth factor. The temperature stable basic fibroblast growth factor is a basic fibroblast growth factor having temperature stability, that is, heat resistance. It is known that natural bFGF is rapidly degraded in a culturing environment of 37°C, but a basic fibroblast growth factor having temperature stability can be produced by introducing a mutation into a part of the amino acid sequence. For example, JP2014-507951A discloses that temperature stability can be imparted by making a mutant in which specific amino acids of bFGF have been substituted (Q65L, Q65I, Q65V, N111A, N111G, C96S, and C96T).

"Always containing 35 ng/mL or more of basic fibroblast growth factor" means that the concentration of basic fibroblast growth factor is always kept at 35 ng/mL or more during the culturing period and that there is no state in which basic fibroblast growth factor is consumed or degraded to be less than 35 ng/mL. In the present invention, a pluripotent stem cell can exhibit good proliferative properties by maintaining the concentration of basic fibroblast growth factor in the medium to be always 35 ng/mL or more, that is, by preventing the concentration of basic fibroblast growth factor in the medium from being less than 35 ng/mL.

The pluripotent stem cell is cultured in a medium always containing basic fibroblast growth factor preferably 40 ng/mL or more, more preferably 50 ng/mL or more, still more preferably 60 ng/mL or more, particularly preferably 70 ng/mL or more, and most preferably 80 ng/mL or more. The upper limit of the concentration of basic fibroblast growth factor in the medium is not particularly limited but is generally 300 ng/ml or less and preferably 200 ng/ml or less. The upper limit of the concentration is more preferably 150 ng/ml or less.

The concentration of basic fibroblast growth factor in the medium can be quantified using a Human bFGF ELISA Kit (Sigma-Aldrich Co., LLC.) by sampling a part of the culture solution of a pluripotent stem cell.

In the present invention, the concentration of the basic fibroblast growth factor during the culturing is preferably maintained at 40% or more, more preferably maintained at 50% or more, still more preferably maintained at 60% or more, particularly preferably maintained at 70% or more, and most preferably maintained at 80% or more of the concentration of the basic fibroblast growth factor at the start of the culturing.

### <Exchange or addition of medium or specific medium component>

In the present invention, exchange or addition of a medium or a specific medium component is preferably not performed for 2 days or more, and may not be performed for 3 days or more, or may not be performed for 4 days or more. In the present invention, a medium or a specific medium component can be exchanged or added preferably at intervals of 2 days or more, more preferably at intervals of 3 days or more, and still more preferably at intervals of 4 days or more. The specific medium component is not particularly limited, but examples thereof include basic fibroblast growth factor.

### <Cell concentration and number of cells >

In the present invention, a pluripotent stem cell is cultured until a cell concentration reaches 1.4 × 10⁶ cells/mL or more. The pluripotent stem cells can be cultured so that the concentration of the cell reaches preferably 1.5 × 10⁶ cells/mL or more, more preferably 1.6 × 10⁶ cells/mL or more, still more preferably 1.8 × 10⁶ cells/mL or more, even still more preferably 2.0 × 10⁶ cells/mL or more, still further preferably 2.3 × 10⁶ cells/mL or more, even still further preferably 2.6 × 10⁶ cells/mL or more, even still further preferably 3.0 × 10⁶ cells/mL or more, and even still further preferably 3.6 × 10⁶ cells/mL or more.

The upper limit of the concentration of the pluripotent stem cell is not particularly limited but is generally 1.0 × 10⁷ cells/mL or less and preferably 5.0 × 10⁶ cells/mL or less.

In the present invention, the culture amount can be appropriately selected according to the required number of pluripotent stem cells.

The cell concentration at the start of the three-dimensional culturing is preferably 1 × 10⁴ cells/mL or more and 1 × 10⁶ cells/mL or less, and more preferably 5 × 10⁴ cells/mL or more and 2 × 10⁵ cells/mL or less.

The cell concentration and the number of cells can be measured by the following method. A part of a cell aggregate in the culture vessel is collected and washed with Dulbecco PBS (D-PBS; PBS means phosphate-buffered saline), TrypLE Select (trade name) is added thereto and treated at 37°C for 5 minutes, cells are separated into single cells, and the cell concentration is measured by a conventional method. The number of cells in the flask can be determined from the culture volume.

### <Proliferation rate>

In the present invention, the number of cells 4 days after the start of the culturing (Day 4) is preferably 5 times or more, more preferably 10 times or more, still more preferably 13 times or more, even still more preferably 14 times or more, still further preferably 20 times or more, even still further preferably 23 times or more, with respect to the number of cells at the start of culturing (Day 0).

In the present invention, the number of cells 8 days after the start of the culturing (Day 8) is preferably 5 times or more, more preferably 100 times or more, still more preferably 200 times or more, even still more preferably 300 times or more, still further preferably 400 times or more, even still further preferably 500 times or more, with respect to the number of cells at the start of culturing (Day 0).

### <Medium>

As a medium used in the present invention, a commercially available medium such as mTeSR1 (registered trademark) (STEMCELL Technologies Inc.) or StemFlex (registered trademark) can be used. However, it is preferable to use StemFlex (registered trademark) since StemFlex contains a temperature stable basic fibroblast growth factor.

Alternatively, for example, Dulbecco Modified Eagle medium (DMEM), a mixed medium of DMEM and F12 (DMEM/F12 = 1:1), and Knockout (trade name) D-MEM (Thermo Fisher Scientific, Inc.) may be used as a basal medium. In a case where the above-described basal medium is used, components such as alternative serum (KSR; Knockout (trade name) Serum Replacement (Thermo Fisher Scientific, Inc.)), fetal bovine serum (FBS), non-essential amino acid (NEAA), L-glutamine, 2-mercaptoethanol, and an antibiotic (for example, streptomycin, penicillin, puromycin, and mitomycin) can be added in any combination to the above basal medium, and a basal medium further added with basic fibroblast growth factor (preferably a temperature stable basic fibroblast growth factor) can be used. As the temperature stable basic fibroblast growth factor, for example, FGF-Basic-TS (Thermostable; manufactured by Proteintech Group Inc.) can be used, but the temperature stable basic fibroblast growth factor is not limited thereto.

The medium to be used in the present invention and satisfying the required conditions of the present invention is preferably a serum-free medium and feeder-free cell medium.

### <Culture condition>

The conditions for culturing the pluripotent stem cell in the present invention are preferably conditions of 37°C and 5% CO₂, and the like but are not particularly limited. In addition, the culturing is preferably culturing with stirring, and the rotation speed of stirring is not particularly limited but is preferably 10 rpm to 100 rpm, more preferably 20 rpm to 60 rpm, and still more preferably 30 rpm to 50 rpm.

The culture vessel used for three-dimensionally culturing a pluripotent stem cell is not particularly limited and any tissue culture vessel can be used. A container having internal surfaces, which are designed such that pluripotent stem cells cultured in the culture vessel do not adhere or attach to the surfaces, may be used.

Three-dimensional culturing of a pluripotent stem cell can be performed in a culturing system in which culture parameters such as temperature, stirring speed, pH, and CO₂ concentration are controlled. Preferably, the control of the culture parameters can be performed automatically using a suitable device. The culture vessel is not particularly limited, but a single-use bioreactor (ABLE corporation) having a volume of 30 mL, a single-use bioreactor having a volume of 100 mL, or the like can be used.

### <Specific example of the present invention>

A pluripotent stem cell (preferably an iPS cell and more preferably a human iPS cell) is cultured on a plate coated with mTeSR1 (registered trademark) (STEMCELL Technologies Inc.) which is a medium for proliferation and Matrigel (registered trademark) at 37°C in an incubator having a concentration of 5% CO₂. The medium is exchanged to a fresh medium every day except the day after seeding. A plate coated with feeder cells may be used instead of the plate coated with Matrigel (registered trademark). As the feeder cell, which is not particularly limited, a mouse embryonic fibroblast (MEF cell) and a mouse embryonic fibroblast (STO cell) can be mentioned.

The pluripotent stem cells cultured by the above method are treated with a protease (for example, TrypLE Select (Thermo Fisher Scientific Ltd.) or the like) to separate the cells into single cells. After measuring the number of cells, the cells are adjusted to have a concentration of 0.2 × 10⁵ cells/mL to 5 × 10⁵ cells/mL and preferably 0.5 × 10⁵ cells/mL to 2 × 10⁵ cells/mL, for example, 1 × 10⁵ cells/mL in a medium (particularly preferably, the StemFlex (registered trademark) medium (Thermo Fisher Scientific Ltd.)) containing bFGF (preferably a temperature stable bFGF). The obtained cell suspension is added to a culture vessel and cultured with stirring. The rotation speed is set to 10 to 100 rpm, and the cells are cultured at 37°C in an incubator having 5% CO₂ concentration.

After the spinner culturing is started as described above, the medium can be exchanged according to a suitable schedule. As described above, in the present invention, exchange or addition of a medium or a specific medium component is preferably not performed for 2 days or more, and may not be performed for 3 days or more, or may not be performed for 4 days or more.

In a case where the medium is exchanged, cell aggregates having the predetermined number of cells (for example, 1.5 × 10⁶ cells) are collected and the medium is removed by centrifugation. After performing treatment with adding an enzyme for enzymatically digesting the cell aggregates of pluripotent stem cells (for example, Gentle Cell Dissociation Reagent (STEMCELL Technologies Inc.)), the treated liquid is put into a filtering device (for example, a 37 µm Reversible Strainer (STEMCELL Technologies Inc.)) at a predetermined speed, and the cell aggregates can be physically fragmented. After removing the supernatant by centrifugation, adding a fresh medium, and resuspending, the cells are adjusted to have a concentration of 0.2 × 10⁵ cells/mL to 5 × 10⁵ cells/mL and preferably 0.5 × 10⁵ cells/mL to 2 × 10⁵ cells/mL, for example, 1 × 10⁵ cells/mL. The culture solution obtained as described above can be added to the culture vessel again and cultured.

### <Maintaining pluripotency>

According to the present invention, a pluripotent stem cell can be proliferated while maintaining pluripotency (that is, in an undifferentiated state).

In the present invention, the pluripotency may be evaluated by monitoring pluripotent stem cells.

The evaluation of pluripotency (or differentiation state) can be performed by, for example, the morphological evaluation and/or the expression analysis ((reverse transcription polymerase chain reaction (RT-PCR), cDNA microarray analysis, flow cytometry, immunohistochemistry, or the like) of pluripotency (undifferentiated state) markers.

For example, RNA can be collected from cells obtained by partially sampling cells during culture, and the gene expression level can be evaluated by real-time quantitative PCR using the cDNA obtained by reverse transcribing the collected RNA into cDNA.

Specific examples of the pluripotency (undifferentiated state) markers (pluripotency-related genes) include NANOG, SOX2, POU5F1, DNMT3B, and LIN28 but are not particularly limited.

Marker names are as follows.
Sox: SRY-boxes
POU5F1: POU domain, class 5, transcription factor 1
DNMT3B: DNA (cytosine-5-)-methyltransferase 3 beta

It has been confirmed, in Examples described later, that the expression levels of the above-mentioned pluripotency (undifferentiated state) markers in the pluripotent stem cell cultured by the method of the present invention are changed only several times in comparison with an undifferentiation control, and thus the expression levels hardly change.

### <Differentiation of pluripotent stem cell>

The pluripotent stem cell cultured by the method of the present invention can be placed under culture conditions suitable for differentiation of the pluripotent stem cell into an embryoid body (EB) after culturing the pluripotent stem cell.

The pluripotent stem cell cultured by the method of the present invention can be taken out from the culture preparation of the pluripotent stem cell and can be subjected to suspension culturing, in the presence of a medium containing serum or a serum replacement and containing no differentiation inhibitory factor, to form EB.

The formation of EB can be monitored according to a conventional method by morphological evaluation (for example, histological staining) and determination of the expression of differentiation-specific markers. The expression of the differentiation-specific marker can be determined by an immunological measurement or an RNA expression analysis (for example, RT-PCR and cDNA microarray).

The cell of EB can be further subjected to culture conditions suitable for differentiation and/or proliferation of a lineage-specific cell to obtain the lineage-specific cell. The "culture conditions suitable for differentiation and/or proliferation of a lineage-specific cell" can be established by a combination of a culturing system (for example, any one of a feeder cell layer, a feeder-free matrix, and suspension culturing) and a medium suitable for differentiation and/or proliferation of a lineage-specific cell. The lineage-specific cell is any type of cells of ectoderm, endoderm, or mesoderm.

Hereinafter, the present invention will be described in more detail with reference to Examples, but the present invention is not limited to Examples.

### Examples

### <Example 1>

### (1) Culturing 1 of iPS cell

A human iPS cell (provided by FUJIFILM Cellular Dynamics, Inc.) was cultured on a plate coated with mTeSR1 (registered trademark) (STEMCELL Technologies Inc.) which is a medium for proliferation and Matrigel (registered trademark) at 37°C in an incubator having a concentration of 5% CO₂. The medium was exchanged to a fresh medium every day except the day after seeding. Subculturing was performed after 4 days of culturing. The subculturing was carried out by treating the cells with 0.5 mmol/L ethylenediaminetetraacetic acid (EDTA) (Thermo Fisher Scientific Ltd.) for about 7 to 8 minutes to strip the cells and then pipetting to separate the stripped cells to a suitable scale. After the subculturing, culturing was continued for 4 days.

### (2) Culturing 2 of iPS cell

TrypLE Select (trade name) (Thermo Fisher Scientific Ltd.) was added to the iPS cells cultured by the above method, and then the cells were treated at 37°C for 5 minutes to separate the cells into single cells. After the number of cells was measured by a conventional method, the cells were adjusted to a concentration of 1× 10⁵ cells/mL in the mTeSR1 (registered trademark) medium containing Y-27632 (FUJIFILM Wako Pure Chemical Corporation) at a final concentration of 1 mmol/L or the StemFlex (registered trademark) medium (Thermo Fisher Scientific Ltd.). It is noted that bFGF contained in the mTeSR1 (registered trademark) medium is a recombinant protein that mimics the sequence of natural type bFGF, and bFGF contained in StemFlex (registered trademark) is a recombinant protein that has been modified to impart temperature stability. 15 mL of a suspension of these cells was added into a single-use bioreactor having a volume of 30 mL (ABLE corporation), and spinner-cultured with a dedicated 6-channel magnetic stirrer. The rotation speed was set to 40 rpm, and the cells are cultured at 37°C in an incubator having 5% CO₂ concentration.

### (3) Culturing 3 of iPS cell

After the spinner culturing was started, all the medium was exchanged according to the schedule shown in Table 1. Four days after the start of the culturing, a part of the cell aggregate in the flask was collected, and the number of cells was counted by the method described below. First, the part of the cell aggregate was washed with Dulbecco PBS (D-PBS; PBS means phosphate-buffered saline) (Thermo Fisher Scientific Ltd.), TrypLE Select (trade name) was added thereto and treated at 37°C for 5 minutes, and cells were separated into single cells. After measuring the cell concentration by a conventional method, the number of cells in the flask was calculated from the culture volume. From the obtained information of the cell concentration, cell aggregates having the number of cells of 1.5 × 10⁶ cells are collected and the medium is removed by centrifugation. After performing treatment for 7 minutes at room temperature with adding Gentle Cell Dissociation Reagent (STEMCELL Technologies Inc.)), the treated liquid is put into a 37 µm Reversible Strainer (STEMCELL Technologies Inc.) at a speed of 1 ml/minute, and the cell aggregates were physically fragmented. After removing the supernatant by centrifugation, 15 mL of the mTeSR1 medium containing Y-27632 at a final concentration of 1 mmol/L or the StemFlex (registered trademark) medium was added thereto and resuspended, and the cells were adjusted to have a concentration of 1× 10⁵ cells/mL. 30 mL of the same culture solution was again added to a single-use bioreactor having a volume of 30 mL, and the cells were cultured in the same manner as in "(2) Culturing 2 of iPS cell" above.

**[Table 1]**

| | Used medium | Day 0 | Day 1 | Day 2 | Day 3 | Day 4 | Day 5 | Day 6 | Day 7 | Day 8 |
|---|---|---|---|---|---|---|---|---|---|---|
| Example 1 | StemFlex (registered trademark) | Culturing start | Medium exchange | Medium Exchange | Medium exchange | Subculturing | Medium exchange | Medium exchange | Medium exchange | Collection Analysis |
| Example 2 | | | | Medium Exchange | | | | Medium exchange | | |
| Example 3 | | | | | | | | | | |
| Comparative Example 1 | mTeSR1 (registered trademark) | | Medium exchange | Medium Exchange | Medium exchange | | Medium exchange | Medium exchange | Medium exchange | |
| Comparative Example 2 | | | | Medium Exchange | | | | Medium exchange | | |
| Comparative Example 3 | | | | | | | | | | |

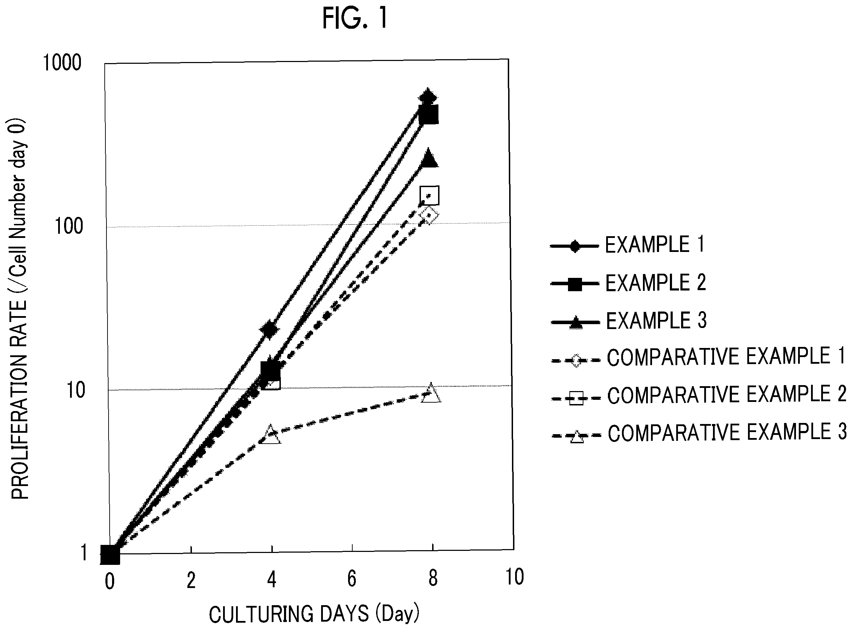

After 8 days of culturing, the cell concentration and the number of cells were measured by the same method as in the above-described "(3) Culturing 3 of iPS cell", and a growth curve was prepared together with the results of the number of cells after 5 days of culturing (Day 4). Fig. 1 shows the results of calculation of the proliferation rate in terms of the relative cell number in a case where the number of cells at the start of the culturing is set to 1, and Table 2 shows the results of changes in cell concentration and cell number during the culturing process. In addition, RNA was collected from partially sampled cells by using RNeasy Micro Kit (Qiagen) and reverse transcribed into cDNA using High Capacity RNA-to-cDNA Kit (Thermo Fisher Scientific Ltd.). The obtained cDNA was evaluated for gene expression level by qPCR using Taqman (registered trademark) Gene Expression Master Mix (Thermo Fisher Scientific Ltd.) and Taqman (registered trademark) Gene Expression Assay (Thermo Fisher Scientific Ltd.) shown in Table 3. The code name of Probe primer set for performing PCR of a certain gene in Taqman (registered trademark) Gene Expression Assay of Thermo Fisher Scientific Ltd. is shown as Assay ID in Table 3. The comparison was performed by the ΔΔCT method (Comparative CT method), and the quantification was performed using iPS cells before the start of the spinner culturing as the reference (undifferentiation control). Further, as a control group deviating from undifferentiated state, iPS cells were cultured in Essential 6 (trade name) medium (Thermo Fisher Scientific Ltd.) for 2 weeks, and cells that promoted spontaneous differentiation were used (differentiation control). The results are shown in Table 4. The numerical value in Table 4 indicates the relative expression level in a case where the gene expression level in the undifferentiation control is set to 1.000.

**[Table 2]**

| | Used medium | Day 4 | | Day 8 | |
|---|---|---|---|---|---|
| | | Cell concentration (x 10⁶ cells/mL) | Cell number (x 10⁶ cells) | Cell concentration (x 10⁶ cells/mL) | Cell number (x 10⁶ cells) |
| Comparative Example 1 | mTeSR1 (registered trademark) | 1.2 | 18.2 | 0.9 | 170.0 |
| Comparative Example 2 | | 1.1 | 17.0 | 1.3 | 224.9 |
| Comparative Example 3 | | 0.5 | 7.9 | 0.2 | 13.9 |
| Example 1 | StemFlex (registered trademark) | 2.3 | 34.4 | 2.6 | 890.0 |
| Example 2 | | 1.3 | 19.3 | 3.6 | 697.8 |
| Example 3 | | 1.4 | 20.9 | 1.8 | 379.6 |

**[Table 3]**

| Gene Symbol | Assay ID |
|---|---|
| NANOG | Hs02387400_g1 |
| SOX2 | Hs00415716_m1 |
| POU5F1 | Hs04260367_gH |
| DNMT3B | Hs00171876_m1 |
| LIN28 | Hs00702808_s1 |

**[Table 4]**

| | NANOG | SOX2 | POU5F1 | DNMT3B | LIN28 |
|---|---|---|---|---|---|
| Example 1 | 1.308 | 0.341 | 1.255 | 1.191 | 1.264 |
| Example 2 | 2.616 | 0.444 | 1.723 | 1.375 | 1.619 |
| Example 3 | 2.404 | 0.206 | 1.313 | 1.453 | 1.456 |
| Comparative Example 1 | 1.055 | 0.479 | 1.155 | 1.253 | 1.151 |
| Comparative Example 2 | 1.376 | 0.573 | 1.363 | 1.717 | 1.344 |
| Comparative Example 3 | 1.515 | 0.376 | 1.251 | 1.704 | 1.324 |
| Undifferentiation control | 1.000 | 1.000 | 1.000 | 1.000 | 1.000 |
| Differentiation control | 0.003 | 17.916 | 0.002 | 0.143 | 0.869 |

From the results in Fig. 1 and Table 2, it has been clear that iPS cells cultured in the mTeSR1 (registered trademark) medium tend to have reduced proliferative properties by reducing the frequency of the medium exchange, while all iPS cells cultured in the StemFlex (registered trademark) medium exhibited good proliferative properties, and Example 3 in which the medium exchange has not been performed has higher proliferative properties than Comparative Example 1 in which the medium was daily exchanged to the mTeSR1 (registered trademark). It has been also found that in the cell aggregate cultured in StemFlex (registered trademark), a cell culture solution having a very high cell concentration of 3.6 × 10⁶ cells/mL at the maximum can be obtained finally.

In addition, from the results in Table 4, it has been confirmed that the expression levels of the pluripotency-related genes (NANOG, SOX2, POU5F1, DNMT3B, and LIN28) in the cell aggregate of iPS cells cultured under the above conditions are changed only several times in comparison with the undifferentiation control, and thus the expression levels hardly change.

From the above results, it has been found that, by suspension-culturing iPS cells in the StemFlex (registered trademark) medium containing bFGF to which temperature stability has been imparted, good proliferative properties are exhibited in a case where the frequency of the medium exchange is reduced, and a cell culture solution having a high cell concentration can be obtained while maintaining the characteristics as the iPS cell.

### <Example 2> Quantification of bFGF in medium

A part of the culture solution of iPS cells cultured by the above-described "(2) Culturing 2 of iPS cell" and "(3) Culturing 3 of iPS cell" was sampled, and Human bFGF ELISA Kit (Sigma-Aldrich Co., LLC.) was used to quantify the amount of bFGF in the medium. The results are shown in Table 2.

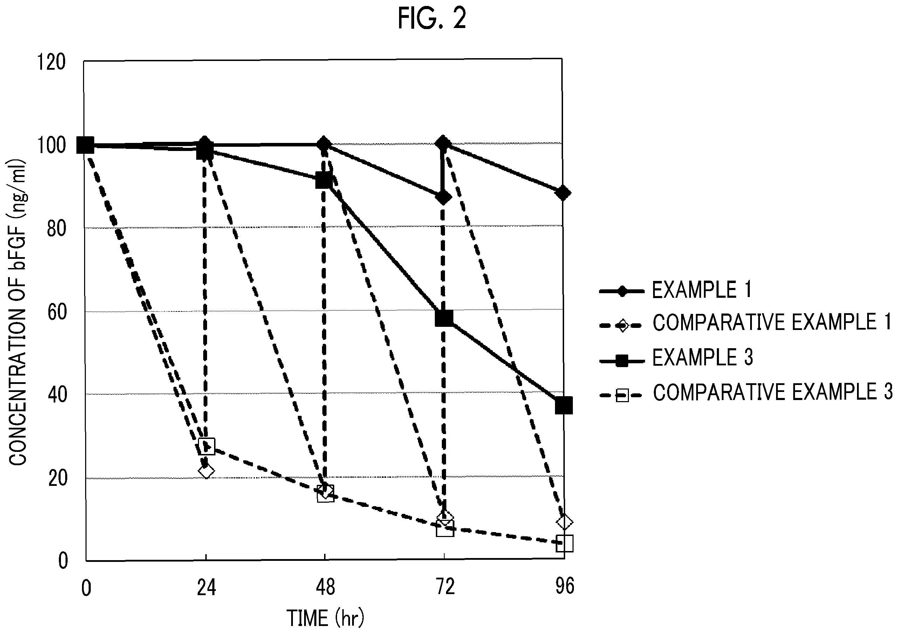

From the results of Fig. 2, it can be seen that the bFGF in mTeSR1 (registered trademark) drops to 20 ng/mL or less in one day of the culturing. This is due to the low stability of natural bFGF in the medium, and it is considered that the iPS cells in the flask cannot always receive the growth stimulation by the bFGF. On the other hand, since bFGF in StemFlex (registered trademark) is always maintained at a concentration of 35 ng/mL or more under any conditions, and it is considered that the iPS cells in the flask are continuously stimulated to proliferate by the bFGF. From the above, it can be seen that maintaining bFGF at a concentration of 35 ng/mL or more during culturing is effective in improving the proliferation of iPS cells.

## Claims

1. A method for three-dimensionally culturing a pluripotent stem cell,
in which a pluripotent stem cell is cultured in a medium that always contains 35 ng/mL or more of basic fibroblast growth factor until a cell concentration reaches 1.4 × 10⁶ cells/mL or more.

2. The method according to claim 1, wherein the basic fibroblast growth factor includes a temperature stable basic fibroblast growth factor.

3. The method according to claim 1 or 2, wherein a concentration of the basic fibroblast growth factor during culturing is maintained at 40% or more of a concentration of the basic fibroblast growth factor at a start of the culturing.

4. The method according to any one of claims 1 to 3, wherein a medium or a specific medium component is exchanged or added at intervals of 2 days or more.

5. The method according to any one of claims 1 to 4, wherein a cell concentration at a start of three-dimensional culturing is 1 × 10⁴ cells/mL or more and 1 × 10⁶ cells/mL or less.

6. The method according to any one of claims 1 to 5, wherein the number of cells 4 days after a start of culturing is 5 times or more the number of cells at the start of the culturing.

7. The method according to any one of claims 1 to 6, wherein the pluripotent stem cell is proliferated while maintaining pluripotency.
